# EUROPEAN PATENT SPECIFICATION

(11) **EP 3 783 097 B1**
(45) Date of publication and mention of the grant of the patent: **22.07.2026**
(21) Application number: 19807067.4
(22) Date of filing: 22.05.2019
(51) Int. Cl.: C12N 5/079, G01N 33/50

(54) **METHOD FOR INDUCING DIFFERENTIATION INTO PLURIPOTENT STEM CELLS- DERIVED CONJUNCTIVAL CELLS**
VERFAHREN ZUR INDUKTION DER DIFFERENZIERUNG IN AUS PLURIPOTENTEN STAMMZELLEN ABGELEITETEN BINDEHAUTZELLEN
PROCÉDÉ POUR INDUIRE LA DIFFÉRENCIATION EN CELLULES CONJONCTIVALES DÉRIVÉES DE CELLULES SOUCHES PLURIPOTENTES

(30) Priority: 23.05.2018 JP 2018098954
(43) Date of publication of application: 24.02.2021
(73) Proprietor: Osaka University, Suita-shi, Osaka 565-0871 (JP)
(72) Inventor: NISHIDA, Kohji, Suita-shi, Osaka 565-0871 (JP); HAYASHI, Ryuhei, Suita-shi, Osaka 565-0871 (JP); NOMI, Kimihito, Suita-shi, Osaka 565-0871 (JP)
(74) Representative: Schön, Christoph
(86) International application number: PCT/JP2019/020364
(87) International publication number: WO 2019/225672

(56) References cited:
- WO-A1-2009/011139
- WO-A1-2016/114285
- JP-A- 2006 129 724
- US-A1- 2018 010 093
- CHUNG ET AL: "Multi-layered culture of primary human conjunctival epithelial cells producing MUC5AC", EXPERIMENTAL EYE RESEARCH, ACADEMIC PRESS LTD, LONDON, vol. 85, no. 2, 26 July 2007 (2007-07-26), pages 226 - 233, XP022169760, ISSN: 0014-4835, DOI: 10.1016/J.EXER.2007.04.005
- RYUHEI HAYASHI ET AL: "Generation of Corneal Epithelial Cells from Induced Pluripotent Stem Cells Derived from Human Dermal Fibroblast and Corneal Limbal Epithelium", PLOS ONE, vol. 7, no. 9, 1 September 2012 (2012-09-01), pages e45435, XP055143151, ISSN: 1932-6203, DOI: 10.1371/journal.pone.0045435
- HIDEYUKI MIYASHITA ET AL: "Long-Term Maintenance of Limbal Epithelial Progenitor Cells Using Rho Kinase Inhibitor and Keratinocyte Growth Factor", STEM CELLS TRANSLATIONAL MEDICINE, vol. 2, no. 10, 1 October 2013 (2013-10-01), US, pages 758 - 765, XP055334387, ISSN: 2157-6564, DOI: 10.5966/sctm.2012-0156
- ROBIN R HODGES ET AL: "Signaling pathways used by EGF to stimulate conjunctival goblet cell secretion", EXPERIMENTAL EYE RESEARCH, ACADEMIC PRESS LTD, LONDON, vol. 103, 26 August 2012 (2012-08-26), pages 99 - 113, XP028516892, ISSN: 0014-4835, [retrieved on 20120904], DOI: 10.1016/J.EXER.2012.08.010
- NOMI KIMIHITO ET AL: "Generation of functional conjunctival epithelium, including goblet cells, from human iPSCs", vol. 34, no. 5, 1 February 2021 (2021-02-01), US, pages 108715, XP055782677, ISSN: 2211-1247, Retrieved from the Internet <URL:https://www.cell.com/cell-reports/pdf/S2211-1247(21)00028-0.pdf> DOI: 10.1016/j.celrep.2021.108715
- HAYASHI, RYUHEI ET AL.: "Co-ordinated ocular development from human iPS cells and recovery of corneal function", NATURE, vol. 531, 2016, pages 376 - 380, XP055383265, DOI: 10.1038/ nature 17000
- HAYASHI, RYUHEI ET AL.: "Coordinated generation of multiple ocular-like cell lineages and fabrication of functional corneal epithelial cell sheets from human iPS cells", NATURE PROTOCOLS, vol. 12, no. 4, 2017, pages 683 - 696, XP055650378, DOI: 10.1038/nprot.2017.007
- BELLEUDI, F. ET AL.: "The receptor tyrosine kinase FGFR2b/KGFR controls early differentiation of human keratinocytes", PLOS ONE, vol. 6, no. 9, 2011, pages e24194, XP055656161, [retrieved on 20190723], DOI: 10.137/journal.pone.0024194

## Description

### TECHNICAL FIELD

The present invention relates to a method for producing conjunctival epithelial cells.

### BACKGROUND ART

The ocular surface is covered with conjunctival and corneal epithelia. The conjunctival epithelium secretes mucins into lacrimal fluid and provides a barrier function for the ocular surface, and the corneal epithelium is engaged in light transmission and refraction, eyeball protection, etc. That is, both epithelia have a crucial role in maintaining ocular surface homeostasis.

Research on human corneal epithelium has been relatively advanced. For example, the present inventors developed a methodology for producing a corneal epithelial cell sheet from iPS cells and confirmed the efficacy of the cell sheet in an animal model to provide a novel therapy for serious corneal diseases such as limbal stem cell deficiency (see Patent Literature 1 and Non Patent Literature 1 and 2).

On the other hand, human conjunctival epithelium is poorly understood. This is due to limited availability of human conjunctival epithelium and difficulty in in vitro culture of conjunctival epithelium.

It is presently known that the conjunctival epithelium in the living body is composed of conjunctival epithelial cells and conjunctival goblet cells and that these cells are derived from conjunctival epithelial stem and progenitor cells located in the basal layer (Non Patent Literature 3).
Non Patent Literature 4 relates to a system for culturing normal human conjunctival epithelial (NHCE) cells under serum-free culture conditions without compromising their ability to differentiate into a mucous epithelium.
Non Patent Literature 5 discloses the induction of corneal epithelial cells from iPS cells by the stromal cell-derived inducing activity (SDIA) differentiation method.
Non Patent Literature 6 discloses that human limbal epithelial cell sheet cultures with KGF and Y-27632 maintain stratification, high expression of both stem/progenitor markers and differentiation markers, and colony-forming cells for a long period of time.
Non Patent Literature 7 is directed to identify signaling pathways that epidermal growth factor (EGF) uses to stimulate mucin secretion from cultured rat conjunctival goblet cells and to compare the pathways used by EGF with those used by the known secretagogue muscarinic, cholinergic agonists.

### CITATION LIST

### Patent Literature

Patent Literature 1: WO 2016/114285

### Non Patent Literature

Non Patent Literature 1:
   Hayashi et al., Nature Protocols, 2017, 12(4), 683-696, doi:10.1038/nprot.2017.007
Non Patent Literature 2:
   Hayashi et al., Nature. 2016 Mar 17, 531, 376-80, doi: 10.1038/nature17000
Non Patent Literature 3:
   Gipson. I.K., Progress in Retinal and Eye Research, 2016, 54,49-63
Non Patent Literature 4:
   CHUNG ET AL, "Multi-layered culture of primary human conjunctival epithelial cells producing MUC5AC", EXPERIMENTAL EYE RESEARCH, ACADEMIC PRESS LTD, LONDON, (20070726), vol. 85 (2007), no. 2, pages 226-233.
Non Patent Literature 5:
   RYUHEI HAYASHI ET AL, "Generation of Corneal Epithelial Cells from Induced Pluripotent Stem Cells Derived from Human Dermal Fibroblast and Corneal Limbal Epithelium", PLOS ONE, (20120901), vol. 7, no. 9, pages 1-10.
Non Patent Literature 6:
   HIDEYUKI MIYASHITA ET AL, "Long-Term Maintenance of Limbal Epithelial Progenitor Cells Using Rho Kinase Inhibitor and Keratinocyte Growth Factor", STEM CELLS TRANSLATIONAL MEDICINE, US, (20131001), vol. 2, no. 10, pages 758-765.
Non Patent Literature 7:
   ROBIN R HODGES ET AL, "Signaling pathways used by EGF to stimulate conjunctival goblet cell secretion", EXPERIMENTAL EYE RESEARCH, ACADEMIC PRESS LTD, LONDON, vol. 103 (2012), pages 99-113.

### SUMMARY OF INVENTION

### TECHNICAL PROBLEM

The detailed mechanism of differentiation of conjunctival epithelial stem and progenitor cells into the above-mentioned two types of cells (conjunctival epithelial cells and conjunctival goblet cell) is unknown. In addition, culture methods for maintaining these two types of cells (particularly conjunctival goblet cells) have not yet been established.

Under current circumstances, conjunctival epithelial studies have to rely on animal experiments due to the difficulty of human cell-based experiments. However, it is not always appropriate to extrapolate the results of animal experiments to humans as they are because the mouse or rabbit conjunctival epithelium is quite different in characteristics from the human counterpart. Recently, mucin-deficient dry eye caused by loss of conjunctival goblet cells has become a problem in real clinical settings, but there are only a limited number of effective therapeutic drugs. Thus, conjunctival epithelial studies have drawn attention from both basic and clinical research fields.

The present invention relates to a method for producing conjunctival epithelial cells as defined in the appended claims.

### SOLUTION TO PROBLEM

The present inventors conducted intensive research to solve the above-mentioned problem. As a result, the present inventors first achieved induction of predominant differentiation into conjunctival epithelial cells, conjunctival goblet cells, and conjunctival epithelial stem and progenitor cells by employing specific culture conditions in the process where multi-zone concentric circular structure (self-formed ectodermal autonomous multi-zone: SEAM) formation is induced according to the method described in Non Patent Literature 1. Based on this finding, the present inventors completed the present invention.

After differentiation, the obtained cell population was stained with three antibodies, namely, antibodies against SSEA-4, CD200, and ITGβ4, and each FACS-sorted fraction was thoroughly analyzed. As a result, the present inventors successfully identified fractions containing the three types of cells, and isolated and enriched these cells. By culturing the isolated cells, the present inventors first achieved in vitro culture of conjunctival epithelial cells together with conjunctival goblet cells.

That is, the present invention relates to the method as defined in the appended claims.

### ADVANTAGEOUS EFFECTS OF INVENTION

The present invention enables the production of a large quantity of conjunctival epithelial cells, conjunctival goblet cells, and conjunctival epithelial stem and progenitor cells from pluripotent stem cells and can greatly contribute to basic and clinical research on the conjunctival epithelium. In addition, functional conjunctival epithelial tissues can be reconstructed, and for example, cell sheet transplantation can provide a radical regenerative therapy and a next-generation therapy for dry eye and other diseases resulting directly from the abnormality of conjunctival cells.

In addition, the conjunctival cells obtained by the present invention are applicable to, for example, drug screening using the conjunctival cells as target cells, which screening has been unfeasible due to limited availability of conjunctival cells.

### BRIEF DESCRIPTION OF DRAWINGS

Fig. 1 shows a typical example of the induction process from iPS cells to conjunctival cells.
Fig. 2 shows an example of the results of sorting of a SEAM cell population.
Fig. 3 shows the results of gene expression analysis of each sorted fraction containing iPS cell-derived cells.
Fig. 4 shows the results of immunofluorescent staining and PAS staining of iPS cell-derived conjunctival goblet cells and conjunctival epithelial cells.
Fig. 5 shows the results of investigation of the fabricability of cell sheets from iPS cell-derived conjunctival epithelial stem and progenitor cells.
Fig. 6 shows the results of gene expression analysis of iPS cell-derived conjunctival epithelial cell sheets and human normal cells.
Fig. 7 shows the results of immunofluorescent staining of iPS cell-derived conjunctival epithelial cell sheets (planar specimen).
Fig. 8 shows the results of immunofluorescent staining of iPS cell-derived conjunctival epithelial cell sheets (sectional specimen).
Fig. 9 shows the results of immunofluorescent staining of iPS cell-derived conjunctival epithelial cell sheets (planar specimen) (conjunctival epithelial cell sheets formed from iPS cell-derived conjunctival epithelial stem and progenitor cells by using EGF signaling activators other than EGF).
Fig. 10 shows the results of gene expression analysis of iPS cell-derived conjunctival epithelial cell sheets (conjunctival epithelial cell sheets matured in a culture medium containing FGF10, an FGFR2b activator other than KGF).

### DESCRIPTION OF EMBODIMENTS

The present inventors produced a cell population mimicking the development of a whole eye from iPS cells (self-formed ectodermal autonomous multi-zone: SEAM), isolated a specific type of progenitor cells from the obtained cell population, and subjecting the progenitor cells to induced differentiation, and if needed, maturation culture to produce a high-purity cell population, for example, corneal epithelial cells etc. For the induction of differentiation into corneal epithelial cells, iPS cells were grown as colonies and cultured in a differentiation medium containing growth factors such as keratinocyte growth factor (KGF) to form a SEAM cell population, and from the SEAM cell population, the desired fraction was isolated and cultured for further differentiation. The obtained cell population contained a high proportion of corneal epithelial cells, and that is why other epithelial cells remained poorly understood. For better understanding, the present inventors conducted intensive research on the method for inducing predominant differentiation into conjunctival epithelial cells. As a result, the present inventors found that, when the formation of the SEAM cell population is induced according to the method described above, culture of iPS cell colonies in the presence of an epidermal growth factor (EGF) signaling activator (hereinafter may be referred to simply as EGF) can induce predominant differentiation into not only conjunctival epithelial cells and conjunctival goblet cells but also conjunctival epithelial stem and progenitor cells in an undifferentiated state. This result demonstrates that iPS cell colonies having a destiny to become various ocular surface epithelial cells of the same lineage can be induced to differentiate into different epithelial cells depending on whether the culture medium contains KGF or EGF. This was first discovered by the present inventors.

Disclosed is a method for inducing differentiation into conjunctival cells, which comprises culturing colonies of pluripotent stem cells in a specific medium in the formation process of a self-formed ectodermal autonomous multi-zone (SEAM) cell population from the pluripotent stem cells. More specifically, pluripotent stem cells are cultured for autonomous differentiation to form colonies and then cultured for differentiation in a medium containing an EGF signaling activator to form a SEAM cell population. From the SEAM cell population, conjunctival epithelial cells, conjunctival goblet cells, and conjunctival epithelial stem and progenitor cells can be isolated using the expression levels of ITGβ4, SSEA-4, and CD200 as indicators as determined with antibodies thereagainst.

The method of the present invention begins with the preparation of a SEAM cell population (colony) from pluripotent stem cells.

The pluripotent stem cells in the present invention are stem cells which have pluripotency, i.e., the ability to differentiate into any type of cells present in a living body, and also have proliferative capacity Examples of the stem cells include embryonic stem cells (ES cells), embryonic stem cells from a non-human cloned embryo obtained by nuclear transfer (non-human ntES cells), spermatogenic stem cells (GS cells), embryonic germ cells (EG cells), induced pluripotent stem cells (iPS cells), and pluripotent cells from cultured fibroblasts or myeloid stem cells (Muse cells) Preferred are ES cells, non-human ntES cells and iPS cells, and more preferred are iPS cells. The pluripotent stem cells are preferably pluripotent stem cells of mammals. The mammal may include humans, mice, rats, cattle, and pigs. With the use of human pluripotent stem cells, a desired cell population that is safe and applicable to human regenerative medicine can be obtained.

The SEAM cell population (colony) is a colony consisting of multiple concentric circular zones of different ectodermal cells, which colony can be obtained by two-dimensional culture of pluripotent stem cells in a serum-free medium without feeder cells.

More specifically, the SEAM cell population can be obtained by, for example, two-dimensional culture of human iPS cells in a known medium appropriate for animal cell culture (e.g., DMEM medium, BME medium, etc.) without crude or unpurified serum. The culture vessel is not particularly limited as long as it can be used for two-dimensional cell culture . The inner surface of the culture vessel is preferably coated with collagen, fibronectin, laminin or laminin fragment, etc. The culture conditions are not particularly limited and can be appropriately determined according to common technical knowledge.

The SEAM cell population (colony) obtained as above is formed as a colony consisting of multiple zones of ectodermal cells generated by cell autonomous differentiation in the absence of exogenous stimuli such as differentiation inducers and differentiation promoters. The colony consists of concentric circular zones of different ectodermal cells, specifically, from the center towards the periphery, the first zone (neuroectodermal lineage cells), the second zone (neural crest lineage cells/optic cup lineage cells), the third zone (ocular surface ectodermal lineage cells), and the fourth zone (surface ectodermal lineage cells). The method for preparing the SEAM cell population may be based on any of the methods described in, for example, Non Patent Literature 1 and 2 and Patent Literature 1.

The four zones of the colony contain different lineage cells, and based on this feature, the cells contained in each particular zone may be isolated and used. Alternatively, the whole colony may be used as it is.

The SEAM cell population (colony) in the present specification is, for example, a cell population obtained by the following procedure. Pluripotent stem cells are seeded at a density of 100 to 700 cells/cm² on laminin 511E8-coated plates, maintained for 8 to 10 days in StemFit (registered trademark) medium (Ajinomoto), and cultured for 4 weeks in a differentiation medium (DM; GMEM (Life Technologies) supplemented with 10% knockout serum replacement (KSR, Life technologies), 1 mM sodium pyruvate (Life Technologies), 0.1 mM non-essential amino acids (Life Technologies), 2 mM L-glutamine (Life Technologies), 1% penicillin-streptomycin solution (Life Technologies) and 55 µM 2-mercaptoethanol (Life Technologies)) for autonomous differentiation.

Next, the obtained SEAM cell population (colony) is cultured in a specific differentiation medium. The medium used in this culture may also be called as a conjunctival differentiation medium.

The conjunctival differentiation medium contains an EGF signaling activator. The EGF signaling activator acts via an EGF receptor. The EGF signaling activator is selected from the group consisting of epidermal growth factor (EGF), transforming growth factor α (TGFα), amphi-regulin (AR), cripto, vaccinia virus growth factor (VVGF), heparin-binding EGF-like growth factor (HB-EGF), and neu differentiation factor/heregulin (NDF/HRG). The concentration of the EGF signaling activator in the medium is usually 0.01 to 1000 ng/mL and preferably 0.1 to 100 ng/mL.

The conjunctival differentiation medium contains a ROCK inhibitor in addition to the EGF signaling activator. The basal medium may be any medium that can be used for epithelial cell culture (serum free medium), and for example, the same one as used in the above-mentioned autonomous differentiation can be used. The "ROCK inhibitor" refers to a substance capable of inhibiting Rho kinase (ROCK: Rho-associated, coiled-coil containing protein kinase). Examples of the ROCK inhibitor that can be used include
N-(4-pyridyl)-4β-[(R)-1-aminoethyl]cyclohexane-1α-carboxami de (Y-27632), Fasudil (HA1077),
(2S)-2-methyl-1-[(4-methyl-5-isoquinolinyl)sulfonyl]hexahyd ro-1H-1,4-diazepine (H-1152),
4β-[(1R)-1-aminoethyl]-N-(4-pyridyl)benzene-1α-carboxamide (Wf-536),
N-(1H-pyrrolo[2,3-b]pyridin-4-yl)-4β-[(R)-1-aminoethyl]cycl ohexane-1α-carboxamide (Y-30141),
N-(3-{[2-(4-amino-1,2,5-oxadiazol-3-yl)-1-ethyl-1H-imidazo[ 4,5-c]pyridin-6-yl]oxy}phenyl)-4-{[2-(4-morpholinyl)ethyl]-oxy}benzamide (GSK269962A), and
N-(6-fluoro-1H-indazol-5-yl)-6-methyl-2-oxo-4-[4-(trifluoro methyl)phenyl]-3,4-dihydro-1H-pyridine-5-carboxamide (GSK429286A).

The culture in the conjunctival differentiation medium is performed in the conditions of 36 to 38°C, preferably 36.5 to 37.5°C, 1 to 25% O₂, and 1 to 15% CO₂. The culture period is at least 1 to 8 weeks, preferably 2 to 6 weeks, and more preferably 3 to 5 weeks. The culture vessel etc. may be the same ones as used in the above-mentioned autonomous differentiation.

Through this culture, ocular surface epithelial stem cells will appear. Next, the ocular surface epithelial stem cells are dissociated by pipetting etc. and cultured in a conjunctival epithelial maintenance medium.

The conjunctival epithelial maintenance medium may be a medium prepared by adding serum replacement to the conjunctival differentiation medium to induce differentiation into conjunctival epithelial progenitor cells. Examples of the "serum replacement" include albumin (e.g., lipid-rich albumin), transferrin, fatty acids, collagen precursors, trace elements (e.g., zinc, selenium), B27 (registered trademark) supplement, and N2 supplement. When B27 supplement is used, the concentration of B27 supplement in the medium is 0.01 to 10 wt%, and preferably 0.5 to 4 wt%.

The culture in the conjunctival epithelial maintenance medium can be performed in the same manner as that in the conjunctival differentiation medium, specifically, in the conditions of 36 to 38°C, preferably 36.5 to 37.5°C, 1 to 25% O₂, and 1 to 15% CO₂. The culture period is at least 3 days to 12 weeks, preferably 1 to 8 weeks, and more preferably 2 to 6 weeks. The culture vessel etc. may be the same ones as used in the above-mentioned autonomous differentiation.

The conjunctival differentiation medium and the conjunctival epithelial maintenance medium may contain various nutrients required for cell maintenance and growth and various components required for differentiation as needed, in addition to the components described above. Examples of the nutrient include carbon sources such as glycerol, glucose, fructose, saccharose, lactose, honey, starch, and dextrin; hydrocarbons such as fatty acids, fats and oils, lecithin, and alcohols; nitrogen sources such as ammonium sulfate, ammonium nitrate, ammonium chloride, urea, and sodium nitrate; inorganic salts such as sodium chloride, potassium salts, phosphoric salts, magnesium salts, calcium salts, iron salts, and manganese salts (e.g., monopotassium phosphate, dipotassium phosphate, magnesium sulfate, sodium chloride, ferrous sulfate, sodium molybdate, sodium tungstate, and manganese sulfate); various vitamins; and amino acids. The amount of each nutrient can be adjusted according to common technical knowledge.

The culture of pluripotent stem cells as described above results in the formation of a SEAM cell population predominantly composed of conjunctival cells, namely, conjunctival epithelial cells, conjunctival goblet cells, and conjunctival epithelial stem and progenitor cells.

From the SEAM cell population obtained by the induction method, conjunctival epithelial cells, conjunctival goblet cells, and conjunctival epithelial stem and progenitor cells can be isolated using the expression levels of ITGβ4, SSEA-4, and CD200 as indicators as determined with antibodies thereagainst. That is, the present invention provides a method for producing conjunctival epithelial cells, conjunctival goblet cells, and conjunctival epithelial stem and progenitor cells.

The method of the present invention for producing of conjunctival epithelial cells, conjunctival goblet cells, and conjunctival epithelial stem and progenitor cells comprises inducing formation of a SEAM cell population predominantly composed of conjunctival epithelial cells, conjunctival goblet cells, and conjunctival epithelial stem and progenitor cells according to the induction method, and isolating desired cells from the SEAM cell population.

Desired cells are isolated from the SEAM cell population using the expression of the three markers as indicators. The isolation can easily be achieved using antibodies specific for the three markers according to the usual method. More specifically, the isolation can be achieved using antibody-labeled magnetic beads, an antibody-immobilized column, or a cell sorter (FACS) with a fluorescent-labeled antibody. The antibody may be obtained commercially or produced in the usual manner. In the isolation method used in the present invention, desired cells are selected based on the expression levels of the markers. Hereinafter, FACS-based selection will be described as an example.

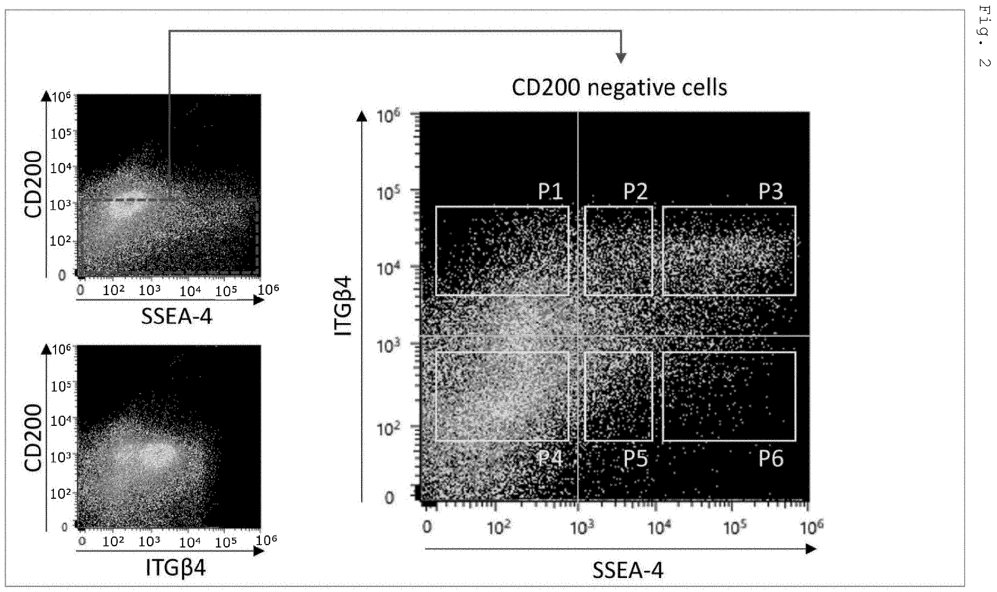

First, CD200 negative selection from the obtained SEAM cell population is performed (left panels in Fig. 2). Next, CD200-negative cells are examined for the expression of ITGβ4 and SSEA-4 (the right panel in Fig. 2, the horizontal axis for SSEA-4 and the vertical axis for ITGβ4). The presence or absence of the expression of each marker is determined, and based on the expression level of each marker, the CD200-negative cells are subdivided into the following six fractions: SSEA-4-negative and ITGβ4-positive cells (P1 fraction), weakly SSEA-4-positive and ITGβ4-positive cells (P2 fraction), SSEA-4-positive and ITGβ4-positive cells (P3 fraction), SSEA-4-negative and ITGβ4-negative cells (P4 fraction), weakly SSEA-4-positive and ITGβ4-negative cells (P5 fraction), and SSEA-4-positive and ITGβ4-negative cells (P6 fraction). In the present invention, from among the six fractions, a combined fraction of weakly SSEA-4-positive and ITGβ4-negative cells (P5 fraction) and SSEA-4-positive and ITGβ4-negative cells (P6 fraction) (these cells are collectively referred to as SSEA-4-positive to weakly SSEA-4-positive and ITGβ4-negative cells (P5 and P6 fractions)) and weakly SSEA-4-positive and ITGβ4-positive cells (P2 fraction) are selected and isolated. The term "weakly positive" means a slightly lower expression level than that in normal cells. In the present invention, the "weakly positive" include an expression level intermediate between those in "positive" and "negative" cells.

The selected SSEA-4-positive to weakly SSEA-4-positive and ITGβ4-negative cells express high levels of MUC5AC (conjunctival goblet cell marker), K13 (conjunctival epithelial cell marker) and PAX6 (ocular cell marker), indicating that the P5 and P6 fractions contain conjunctival epithelial cells and conjunctival goblet cells derived from iPS cells.

The weakly SSEA-4-positive and ITGβ4-positive cells express a high level of p63 (epithelial stem cell marker) and express moderate levels of K13 (conjunctival epithelial cell marker) and PAX6 (ocular cell marker), indicating that the P2 fraction contains conjunctival epithelial stem and progenitor cells derived from iPS cells.

The cells isolated above can be directly cultured in a known culture medium. For example, the cells may be cultured in the above-mentioned conjunctival differentiation medium or conjunctival epithelial maintenance medium or other culture media containing different growth factors.

The obtained cells can be used for various applications. More specifically, for example, the cells in the P5 and P6 fractions, which are in a differentiated state, can be applied to basic research such as detailed analysis of conjunctival epithelial cells and conjunctival goblet cells, the examination of optimal culture conditions of these cells, and drug screening tests. The cells in the P2 fraction, which can be induced to differentiate into conjunctival epithelial cell and conjunctival goblet cells in culture, can be used for the production of conjunctival epithelial cell sheets for transplantation and the examination of optimal culture conditions of conjunctival epithelial tissues. Further disclosed but not part of the present invention is a method for producing a conjunctival epithelial cell sheet, which method comprises the step of culturing conjunctival epithelial stem and progenitor cells obtained by the induction method. A specific embodiment, which is not part of the present invention is, for example, a method for producing a conjunctival epithelial cell sheet for transplantation, which method comprises the step of culturing conjunctival epithelial stem and progenitor cells obtained by the induction method. The obtained conjunctival epithelial cell sheet is promising to be applied to the treatment of eye diseases related to conjunctival epithelial dysfunction (e.g., mucin-deficient dry eye). The growth factor contained in the medium for culturing the conjunctival epithelial stem and progenitor cells is not particularly limited. Preferably, at least KGF is contained in the medium. In addition, a fibroblast growth factor receptor 2b (FGFR2b) activator (e.g., FGF10 etc.) may be contained in the medium. The concentration of the growth factor in the medium is usually 0.1 to 1000 ng/mL and preferably 1 to 100 ng/mL.

Further disclosed, but not part of the present invention is a screening method using conjunctival epithelial cells and conjunctival goblet cells obtained by the induction method or using a conjunctival epithelial tissue composed of conjunctival epithelial cells and conjunctival goblet cells derived from conjunctival epithelial stem and progenitor cells obtained by the induction method. In an embodiment of the screening method, the expression of a functional protein-coding gene or the activity of a functional protein in the conjunctival epithelial tissue is used as an indicator, and a substance capable of promoting the expression or activity is determined as a candidate compound for the treatment of a conjunctiva-related disease. More specifically, the expression level of a functional protein-coding gene or the activity level of a functional protein is measured in the conjunctival epithelial tissue in contact with a test substance, and when the expression level or the activity level is higher than that in control cells not in contact with the test substance, the test substance is determined as a candidate compound for the treatment of a conjunctiva-related disease.

### EXAMPLES

Hereinafter, the present invention will be described in detail with reference to examples. Henceforth, "room temperature" refers to 25°C.

### Example 1

### Induction of differentiation into ocular surface stem cells

For induced differentiation of iPS cells into ocular surface stem cells, the protocol for self-formed ectodermal autonomous multi-zone (SEAM) formation (Hayashi et al. Nature. 2016 Mar 17;531(7594):376-80., Hayashi et al., Nature Protocols, 2017, 12(4), 683-696, doi: 10.1038/nprot.2017.007) was used.

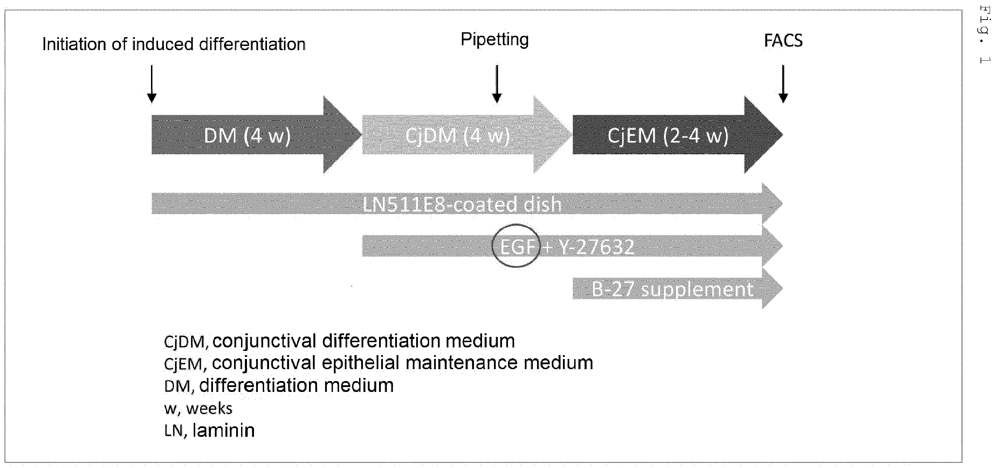

The specific procedure was as follows. Human iPS cells (201B7) were seeded at a density of 300 to 700 cells/cm² on laminin 511E8-coated plates, maintained for 8 to 10 days in StemFit (registered trademark) medium (Ajinomoto), and cultured for 4 weeks in a differentiation medium (DM; GMEM (Life Technologies) supplemented with 10% knockout serum replacement (KSR, Life technologies), 1 mM sodium pyruvate (Life Technologies), 0.1 mM non-essential amino acids (Life Technologies), 2 mM L-glutamine (Life Technologies), 1% penicillin-streptomycin solution (Life Technologies) and 55 µM 2-mercaptoethanol (Life Technologies)). After that, culture was continued for 4 weeks in conjunctival differentiation medium (CjDM; a 1:1 (v/v) mixed medium of DM and Cnt-20 or Cnt-PR (without EGF or FGF2, CeLLnTEC Advanced Cell Systems) supplemented with 10 ng/mL EGF (R&D Systems), 10 µM Y-27632 (Wako) and 1% penicillin-streptomycin solution) and subsequently for additional about 2 to 4 weeks in conjunctival epithelial maintenance medium (CjEM; DMEM/F12 (2:1 (v/v)) (Life Technologies) supplemented with 2% B27 supplement (Life Technologies), 1% penicillin-streptomycin solution, 10 ng/mL EGF and 10 µM Y-27632) for induced differentiation. The time schedule is shown in Fig. 1.

### Example 2

### Sorting out of desired cells by FACS

From the SEAM cell population obtained in Example 1, CD200-negative cells were sorted out using cell sorter SH800 (SONY), plotted based on SSEA-4 versus ITGβ4, and subdivided into the six fractions shown in Fig. 2. These six fractions were designated as SSEA-4-negative and ITGβ4-positive cells (P1 fraction), weakly SSEA-4-positive and ITGβ4-positive cells (P2 fraction), SSEA-4-positive and ITGβ4-positive cells (P3 fraction), SSEA-4-negative and ITGβ4-negative cells (P4 fraction), weakly SSEA-4-positive and ITGβ4-negative cells (P5 fraction), and SSEA-4-positive and ITGβ4-negative cells (P6 fraction).

### Example 3

### Gene expression analysis

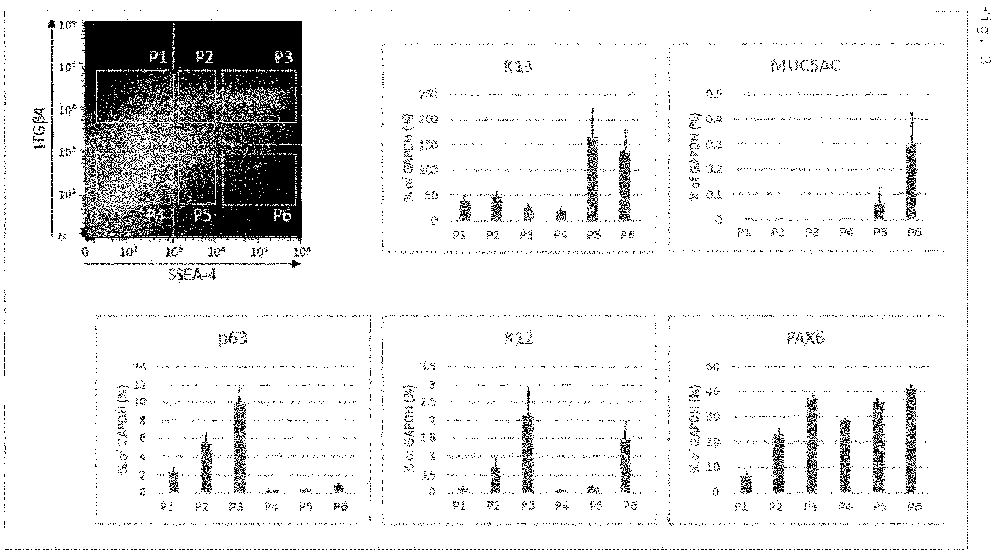

RNA was extracted from each of the six fractions obtained in Example 2 using QIAzol Lysis Reagent (QIAGEN) and subjected to RT-qPCR to examine the expression of specific markers. The results are shown in Fig. 3. In the figure, the results are shown for the cells in each fraction (n = 3 for the P4 fraction and n = 5 for the other fractions), and the error bars represent the standard error of the mean.

As shown in Fig. 3, the cells in the P2 fraction expressed a high level of p63 (epithelial stem cell marker), expressed moderate levels of K13 (conjunctival epithelial cell marker) and PAX6 (ocular cell marker), and expressed a low level of K12 (corneal epithelial cell marker). These results demonstrate that the cells show the phenotype of conjunctival epithelial stem and progenitor cells. Also shown is that the cells in the P5 and P6 fractions expressed high levels of MUC5AC (conjunctival goblet cell marker), K13 and PAX6 and expressed a low level of p63. These results demonstrate that the cells show the phenotypes of conjunctival goblet cells and conjunctival epithelial cells.

### Example 4

### Immunofluorescent staining

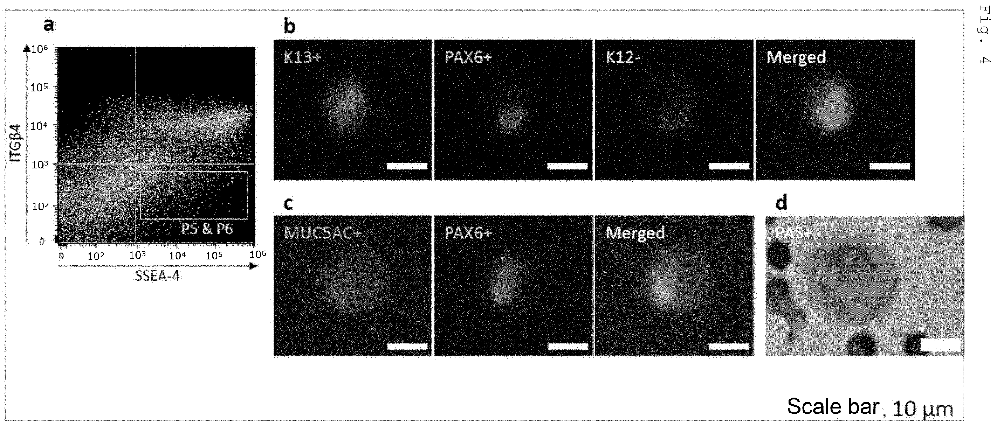

The cells in the P5 and 6 fractions sorted out in Example 2 were fixed and permeabilized with a BD Cytofix/Cytoperm (registered trademark) Kit (554714, BD Biosciences) and suspended at 50,000 cells/mL in DMEM/F12 medium. The cell suspension was loaded at 200 µL per column and centrifuged with Cytospin (A78300003, Thermo Fisher Scientific) to prepare cell specimens on slides. Each specimen was blocked with 5% NST (TBS supplemented with 5% Normal Donkey Serum and 0.3% Triton; T903, TaKaRa Bio) at room temperature for 1 hour and subjected to primary antibody reaction at 4°C overnight using an anti-MUC5AC antibody (1:200; sc-21701, Santa Cruz Biotechnology), an anti-K13 antibody (1:200; ab16112, abcam), an anti-K12 antibody (1:200; sc-17098, Santa Cruz Biotechnology), or an anti-PAX6 antibody (1:300; PRB-278P, COVANCE). After washing with TBS twice for 5 minutes each, treatment with the corresponding secondary antibody labeled with Alexa Fluor 488, Alexa Fluor 568, or Alexa Fluor 647 (1:200; Life Technologies) and staining with Hoechst 33342 (1:100; H3570, Molecular Probes) were performed at room temperature for 1 hour. Each specimen was observed with a fluorescence microscope. The results are shown in Fig. 4.

Separately, the cells in the P5 and P6 fractions were fixed and permeabilized with a BD Cytofix/Cytoperm (registered trademark) Kit. The cells were stained with a PAS staining kit (101646, Merck KGaA) and observed with an inverted microscope. The results are shown in Fig. 4.

As shown in Fig. 4, conjunctival epithelial cells, which were K13+, K12-, and PAX6+ cells (Fig. 4b), and conjunctival goblet cells, which were MUC5AC+ and PAX6+ cells (Fig. 4c), were observed in the P5 and P6 fractions. PAS+ cells (Fig. 4d) were also observed.

### Example 5

### Culture of human iPS cell-derived conjunctival epithelial stem and progenitor cells

The cells in the P2 fraction obtained in the same manner as in Example 2 were cultured on cell culture inserts coated with a laminin 511E8 fragment (0.5 µg/cm²) for 2 to 3 weeks. The culture medium used was a KGFmedium (DMEM/F-12 supplemented with 10 ng/mL KGF (Wako), 10 µM Y-27632, 2% B27 supplement, and 1% penicillin-streptomycin solution) or an EGF medium (DMEM/F-12 supplemented with 10 ng/mL EGF, 10 µM Y-27632, 2% B27 supplement, and 1% penicillin-streptomycin solution).

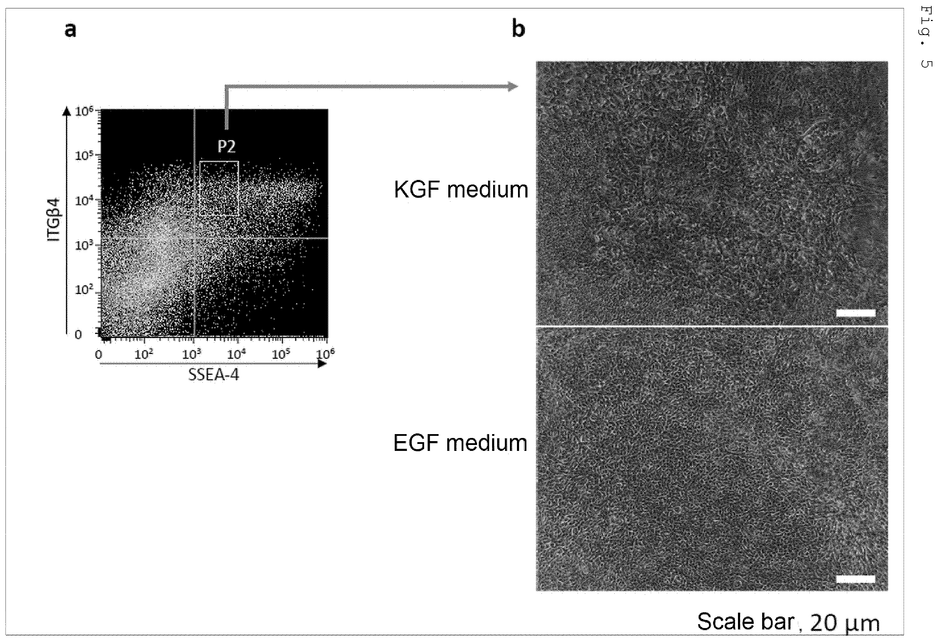

As shown in phase-contrast micrographs (Fig. 5b), cell sheets were successfully formed from the cells in the P2 fraction sorted out by FACS (Fig. 5a) in both culture conditions.

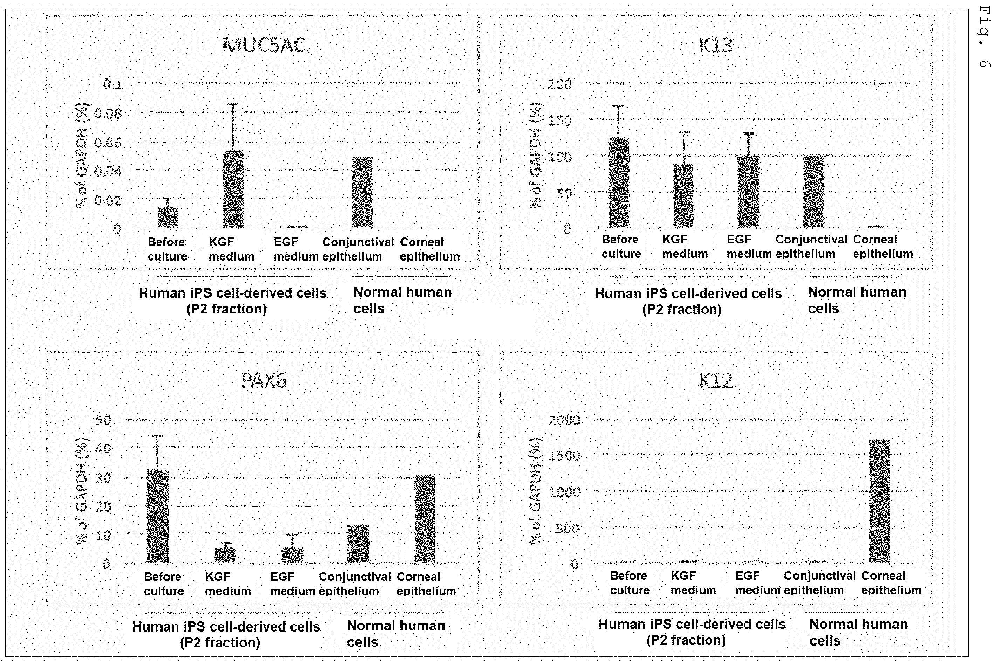

The cells in the P2 fraction and normal human cells before and after culture were subjected to gene expression analysis in a similar manner to Example 3. Only the cells in the P2 fraction cultured in the KGF medium showed an increased MUC5AC expression as compared with that before culture. In addition, the cells in the P2 fraction expressed a high level of K13, a moderate level of PAX6, and a low level of K12, showing the same phenotype as that of normal human conjunctival cells (Fig. 6) . In the figure, the results are shown for the cells in the P2 fraction before culture (n = 5), the cells in the P2 fraction after culture (n = 4), and normal human cells (n = 1), and the error bars represent the standard error of the mean.

Next, the cell sheets formed from the cells in the P2 fraction were harvested from the cell culture inserts and fixed with methanol (131-01826, Wako) at room temperature for 20 minutes for preparation of planar specimens. The specimens were stained. For preparation of sectional specimens, the cell sheets were embedded in O.C.T. compound (4583, Tissue-Tek), stored at -80°C, cryosectioned at a thickness of 5 µm with a cryostat, dried at room temperature for 30 minutes or more, and fixed with methanol at room temperature for 20 minutes. The specimens were stained. For staining, the specimens were blocked with 5% NST at room temperature for 1 hour and subjected to primary antibody reaction at 4°C overnight using an anti-MUC5AC antibody (1:200), an anti-K13 antibody (1:200), an anti-K12 antibody (1:200), or an anti-PAX6 antibody (1:300). After washing with TBS twice for 5 minutes each, treatment with the corresponding secondary antibody labeled with Alexa Fluor 488 or Alexa Fluor 568 (1:200) and staining with Hoechst 33342 (1:100) were performed at room temperature for 1 hour. Each specimen was observed with a fluorescence microscope.

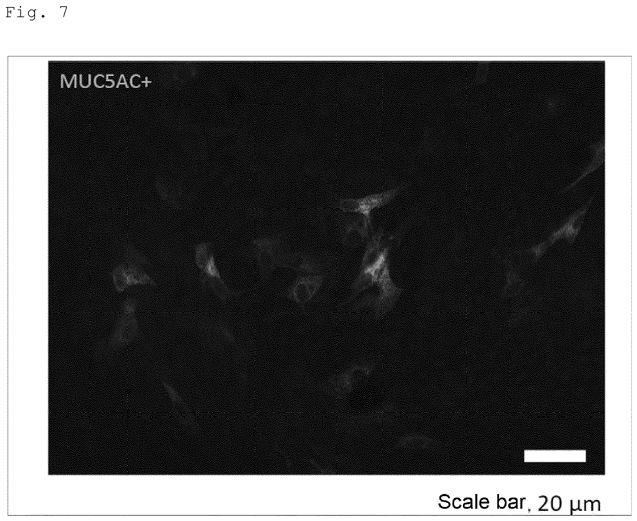

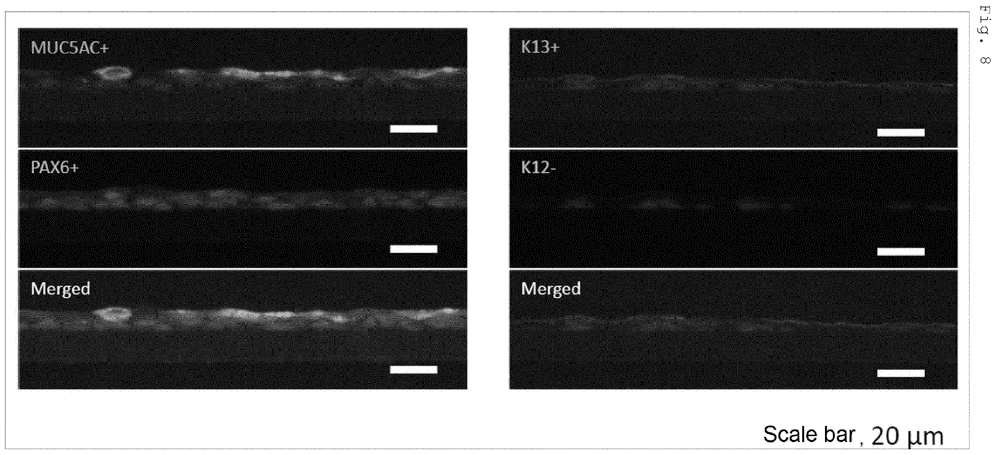

The immunofluorescent staining of the planar specimens confirmed that MUC5AC+ cells were present only after culturing in the KGF medium (Fig. 7). The immunofluorescent staining of the sectional specimens confirmed that MUC5AC+, K13+, PAX6+, and K12- cells, which have the same phenotype as that of conjunctival epithelial cells, were present only after culturing in the KGF medium (Fig. 8).

### Example 6

### Examination of EGF signaling activators

Differentiation into ocular surface stem cells was induced as described in Example 1 except that the conjunctival differentiation medium contained transforming growth factorα (TGFα) or amphi-regulin (AR) instead of EGF. After that, the cells in the P2 fraction were sorted out as described in Example 2 and cultured in the KGF medium as described in Example 5. The obtained cell sheet was stained and examined for MUC5AC expression.

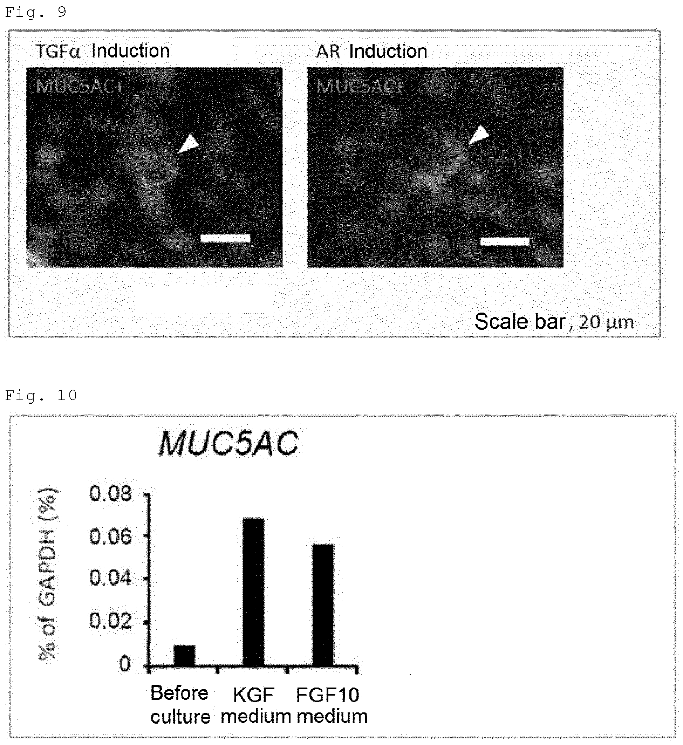

The results show that conjunctival epithelial cell sheets containing MUC5AC-positive cells were successfully formed also when EGF signaling activators other than EGF were used (Fig. 9).

### Example 7

### Examination of growth factor for conjunctival epithelial stem and progenitor cells

The cells in the P2 fraction were cultured as described in Example 5 except that the culture medium for conjunctival epithelial stem and progenitor cells contained an FGFR2b activator (FGF10) instead of KGF. The obtained cell sheets were subjected to RT-qPCR as described in Example 3, and the expression level of MUC5AC was examined.

The results show that conjunctival epithelial cell sheets expressing MUC5AC were successfully formed also when FGF10 was used (Fig. 10).

### INDUSTRIAL APPLICABILITY

The present invention enables differentiation of pluripotent stem cells including iPS cells into conjunctival epithelial cells, conjunctival goblet cells, and conjunctival epithelial stem and progenitor cells and therefore is very useful for, for example, basic research on conjunctival epithelial cells and conjunctival goblet cells, regenerative therapy for intractable ocular surface diseases, and research related to the diseases.

## Claims

1. A method for producing conjunctival epithelial cells, conjunctival goblet cells, and conjunctival epithelial stem and progenitor cells, the method comprising
obtaining a self-formed ectodermal autonomous multi-zone (SEAM) cell population colony consisting of multiple zones of ectodermal cells generated by cell autonomous differentiation of pluripotent stem cells in a medium appropriate for animal cell culture in the absence of differentiation inducers and differentiation promoters,
culturing the colony of the SEAM cell population in a conjunctival differentiation medium containing an epidermal growth factor (EGF) signaling activator selected from the group consisting of epidermal growth factor (EGF), transforming growth factor α (TGFα), amphi-regulin (AR), cripto, vaccinia virus growth factor (VVGF), heparin-binding EGF-like growth factor (HB-EGF), and neu differentiation factor/heregulin (NDF/HRG)and a substance capable of inhibiting Rho kinase (ROCK inhibitor)and
isolating cells from the SEAM cell population using the expression levels of ITGβ4, SSEA-4, and CD200 as indicators as determined with antibodies thereagainst.

2. The method according to claim 1, wherein the isolated cells are CD200-negative cells.

3. The method according to claim 2, wherein the isolated cells are SSEA-4 weakly positive and ITGβ4 positive conjunctival epithelial stem and progenitor cells, wherein weakly positive corresponds to an intermediate expression level between those in positive and negative cells.

4. The method according to claim 2, wherein the isolated cells are SSEA-4 weakly positive or positive and ITGβ4-negative conjunctival epithelial cells and/or conjunctival goblet cells, wherein weakly positive corresponds to an intermediate expression level between those in positive and negative cells.

## Patentansprüche

1. Verfahren zur Herstellung von Bindehautepithelzellen, Bindehautbecherzellen und Bindehautepithel-Stamm- und -Vorläuferzellen, wobei das Verfahren Folgendes umfasst:
Erhalten einer selbstgebildeten ektodermalen autonomen Mehrzonen- (SEAM)-Zellpopulationskolonie, die aus mehreren Zonen ektodermaler Zellen besteht, die durch autonome Zelldifferenzierung von pluripotenten Stammzellen in einem Medium, das für eine Tierzell-Kultur geeignet ist, in Abwesenheit von Differenzierungsinduktoren und Differenzierungspromotoren erzeugt werden,
Kultivieren der Kolonie der Population der SEAM-Zellen in einem Medium für Bindehautdifferenzierung, das einen epidermaler Wachstumsfaktor (EGF)-Signalaktivator, der aus der Gruppe ausgewählt ist, die aus epidermalem Wachstumsfaktor (EGF), transformierendem Wachstumsfaktor α (TGFα), Amphi-Regulin (AR), Cripto, Vaccinia-Virus-Wachstumsfaktor (VVGF), Heparin-bindendem EGF-ähnlichem Wachstumsfaktor (HB-EGF) und Neu-Differenzierungs-Faktor/Heregulin (NDF/HRG) besteht, sowie eine Substanz, die in der Lage ist, Rho-Kinase zu hemmen (ROCK-Inhibitor), enthält, und
Isolieren der Zellen aus der SEAM-Zellpopulation unter Verwendung der Expressionsniveaus von ITGβ4, SSEA-4 und CD200 als Indikatoren gemäß Bestimmung mit Antikörpern gegen diese.

2. Verfahren gemäß Anspruch 1, wobei es sich bei den isolierten Zellen um CD200-negative Zellen handelt.

3. Verfahren gemäß Anspruch 2, wobei es sich bei den isolierten Zellen um schwach SSEA-4-positive und ITGβ4-positive Bindehautepithelpithel-Stamm- und -Vorläuferzellen handelt, wobei "schwach positiv" einem mittleren Expressionsniveau zwischen denjenigen in positiven und negativen Zellen entspricht.

4. Verfahren gemäß Anspruch 2, wobei es sich bei den isolierten Zellen um schwach SSEA-4-positive oder SSEA-4-positive und ITGβ4-negative Bindehautepithelzellen und/oder Bindehautbecherzellen handelt, wobei "schwach positiv" einem mittleren Expressionsniveau zwischen denjenigen in positiven und negativen Zellen entspricht.

## Revendications

1. Procédé de production de cellules épithéliales conjonctivales, de cellules caliciformes conjonctivales et de cellules souches et progénitrices épithéliales conjonctivales, le procédé comprenant
l'obtention d'une colonie de population cellulaire multizones autonome ectodermique auto-formée (SEAM), constituée de multiples zones de cellules ectodermiques générées par différenciation cellulaire autonome de cellules souches pluripotentes dans un milieu approprié à la culture de cellules animales, en l'absence d'inducteurs de différenciation et de promoteurs de différenciation,
la culture de la colonie de la population cellulaire SEAM dans un milieu de différenciation conjonctivale contenant un activateur de la signalisation du facteur de croissance épidermique (EGF) choisi parmi le groupe comprenant le facteur de croissance épidermique (EGF), le facteur de croissance transformant α (TGFα), l'amphi-réguline (AR), le cripto, le facteur de croissance du virus de la vaccine (VVGF), le facteur de croissance de type EGF se liant à l'héparine (HB-EGF) et le facteur de différenciation neu/héréguline (NDF/HRG), ainsi qu'une substance capable d'inhiber la Rho kinase (inhibiteur de ROCK) et
l'isolement des cellules de la population cellulaire SEAM à l'aide des niveaux d'expression de l'ITGβ4, du SSEA-4 et du CD200 comme indicateurs, tels que déterminés à l'aide d'anticorps dirigés contre ceux-ci.

2. Procédé selon la revendication 1, dans lequel les cellules isolées sont des cellules CD200-négatives.

3. Procédé selon la revendication 2, dans lequel les cellules isolées sont des cellules souches et progénitrices épithéliales conjonctivales SSEA-4 faiblement positives et ITGβ4 positives, dans lequel le terme « faiblement positif » correspond à un niveau d'expression intermédiaire entre ceux des cellules positives et négatives.

4. Procédé selon la revendication 2, dans lequel les cellules isolées sont des cellules épithéliales conjonctivales et/ou des cellules caliciformes conjonctivales SSEA-4 faiblement positives ou positives et ITGβ4-négatives, dans lequel le terme « faiblement positif » correspond à un niveau d'expression intermédiaire entre ceux des cellules positives et négatives.
